# EUROPEAN PATENT SPECIFICATION

(11) **EP 4 306 103 B1**
(45) Date of publication and mention of the grant of the patent: **24.12.2025**
(21) Application number: 22767446.2
(22) Date of filing: 07.03.2022
(51) Int. Cl.: A61K 9/08, A61K 9/00, A61K 47/22, A61K 47/18, A61K 47/20, A61K 47/10, A61K 38/00, A61P 37/08, C07K 14/735

(54) **FORMULATION OF FUSION PROTEIN INCLUDING EXTRACELLULAR DOMAIN OF ALPHA SUBUNIT OF IGE FC RECEPTOR**
FORMULIERUNG EINES FUSIONSPROTEINS MIT EXTRAZELLULÄRER DOMÄNE DER ALPHA-UNTEREINHEIT DES IGE-FC-REZEPTORS
FORMULATION DE PROTÉINE DE FUSION COMPRENANT LE DOMAINE EXTRACELLULAIRE DE SOUS-UNITÉ ALPHA DU RÉCEPTEUR FC D'IGE

(30) Priority: 09.03.2021 KR 20210030501
(43) Date of publication of application: 17.01.2024
(60) Divisional application: 25217213.5
(73) Proprietor: GI Innovation, Inc., Seoul 05855 (KR)
(72) Inventor: JANG, Myung Ho, Seoul 05849 (KR); CHO, Young-Gyu, Daejeon 34032 (KR); KWON, Soontak, Bucheon-si Gyeonggi-do 14539 (KR)
(74) Representative: HGF
(86) International application number: PCT/KR2022/003207
(87) International publication number: WO 2022/191550

(56) References cited:
- WO-A1-2020/097141
- WO-A1-2021/006599
- KR-A- 20120 103 702
- KR-B1- 102 038 672
- KR-B1- 102 038 675
- US-A1- 2020 353 049
- ANN L DAUGHERTY AND RANDALL J MRSNY ED - STEVEN J SHIRE ET AL: "Formulation and Delivery Issues for Monoclonal Antibody Therapeutics", 1 January 2010, CURRENT TRENDS IN MONOCLONAL ANTIBODY DEVELOPMENT AND MANUFACTURING, SPRINGER, US, PAGE(S) 103 - 129, ISBN: 978-0-387-76642-3, XP009133774
- WANG W ET AL: "ANTIBODY STRUCTURE, INSTABILITY, AND FORMULATION", JOURNAL OF PHARMACEUTICAL SCIENCES, WILEY, vol. 96, no. 1, 1 January 2007 (2007-01-01), pages 1 - 26, XP009084505, ISSN: 0022-3549, DOI: 10.1002/JPS.20727

## Description

### Technical Field

The present invention relates to a formulation optimized for a fusion protein dimer, comprising an extracellular domain of an alpha subunit of an IgE Fc receptor.

### Background Art

Allergic diseases, such as allergic rhinitis, atopic dermatitis, and food allergy, including asthma are increasing rapidly in industrialized and westernized modern societies, and the occurrence of anaphylaxis, a severe allergic disease, is also increasing. These chronic immune diseases severely impair individual's quality of life and socioeconomic costs are soaring accordingly. Thus, there is a desperate need for measures to overcome such diseases.

Most allergic diseases are caused by an excessive immune response of immunoglobulin E (IgE). IgE is an antibody that is present in serum at a very low concentration under a normal condition. IgE is also usually produced by innocuous antigens. There is a case where the number of IgE is increased without any particular stimulus. Such a case may lead to allergic diseases. The abnormally increased number of IgE can bind to the high affinity IgE Fc receptor (FcεRI) which are expressed on the surface of mast cells, basophils and the like. Such binding causes mast cells or basophils to release chemical mediators such as histamine, leukotriene, prostaglandin, bradykinin and platelet-activating factor. Release of these chemical mediators results in allergic symptoms. In particular, allergic diseases may exhibit worsened symptoms due to the binding between IgE and FcεRI.

Currently, various methods, such as allergen avoidance, administration of anti-allergic drugs, modulation of IgE synthesis in the body, and development of anti-IgE antibodies, have been proposed to treat allergic diseases. However, therapeutic methods known so far have many drawbacks, such as inability to solve underlying cause of allergy, insufficient drug efficacy, and occurrence of serious side effects.

In addition, immunoglobulin compositions capable of binding IgE and FcγRIIb with high affinity and inhibiting IgE-expressing cells are being studied (KR 10-1783272 B1). Such compositions have been reported to be useful for treating IgE-mediated disorders, including allergies and asthma. Patent document WO 2021/006599 discloses compositions comprising a polypeptide dimer comprising two monomers containing the extracellular domain (FcεRIa-ECD) of the alpha subunit of the IgE Fc receptor.

Meanwhile, Omalizumab (trade name: Xolair), which targets the Fc portion of an IgE antibody, has been developed and is being used as a therapeutic agent for refractory severe asthma and refractory urticaria. However, a high-dose administration of omalizumab to maintain therapeutic effects leads to a high cost burden, and side effects such as angioedema and anaphylactic reaction. In addition, from the post-marketing results, allergic granulomatous vasculitis and idiopathic severe thrombocytopenia have been reported. Accordingly, there is growing need for development of therapeutic agents capable of effectively treating allergic diseases without side effects.

In order to develop a therapeutic agent that may efficiently treat allergic diseases without side effects, a fusion protein comprising an extracellular domain of an alpha subunit of an IgE Fc receptor was developed, and it was confirmed that the fusion protein may treat allergic diseases (KR 10-2038675 B1). In order to efficiently apply this protein to the treatment of allergic diseases, it is necessary to develop a stable high-concentration protein formulation that provides dosing and administration advantages.

### Detailed Description of Invention

### Technical Problem

An object of the present invention is to provide an optimized formulation to commercialize a fusion protein dimer for the treatment of allergic diseases mediated by IgE. Specifically, the present inventors developed a high-concentration aqueous pharmaceutical formulation optimized for subcutaneous injection to commercialize the fusion protein dimer, thus have completed the present invention.

### Solution to Problem

In order to solve the above problems, the present invention provides an aqueous pharmaceutical formulation according to the claims comprising a fusion protein dimer comprising an extracellular domain of an alpha subunit of an IgE Fc receptor (FcεRIα ECD) and having a pH of 6.0 to 7.0.

### Effects of Invention

The aqueous pharmaceutical formulation according to the present invention, which comprises a fusion protein dimer comprising an extracellular domain of an alpha subunit of an IgE Fc receptor (FcεRIα ECD) and having a pH of 6.0 to 7.0, has excellent stability. Moreover, the formulation according to the present invention comprising the fusion protein dimer at a high concentration can be administered quickly and conveniently by subcutaneous injection. In addition, the formulation has excellent stability and can increase the storage period by reducing aggregate formation. Therefore, the formulation comprising a fusion protein dimer comprising an extracellular domain of an alpha subunit of an IgE Fc receptor (FcεRIα ECD) can be usefully utilized as an injection for the treatment of allergic diseases.

### Brief Description of Drawings

FIG. 1 is a photograph of the samples (#1 to #4) that were subjected to light stress for 7.5 hours under a condition of 750 W/m².
FIG. 2 is a photograph of the samples (#1 to #6) that were subjected to stress for 5 days under a condition of 35°C and 200 rpm.
FIG. 3 is a photograph of the samples (#1 to #6) that were subjected to stress for 14 days under a condition of 35°C and 200 rpm.
FIG. 4 is a graph showing the recovery (%) of the samples (#1 to #6) that were subjected to stress for 14 days under a condition of 35°C and 200 rpm.
FIG. 5 is a graph showing the main peak areas of the samples according to each stress condition.
FIG. 6 is a graph showing the aggregates area of the samples according to each stress condition.
FIG. 7 is a graph showing the fragments area of the samples according to each stress condition.
FIG. 8 is a photograph of a syringe filled with the sample (#1 or #2) in the initial state and a syringe after stress exposure for 14 days at 35°C and 200 rpm.
FIG. 9 is a graph showing a force/length diagram. In this case, it was performed at the push-out speed of 190 mm/min.
FIG. 10 is a graph showing the main peak areas of the samples (#1 and #2) according to each stress condition.

### Best Mode for Carrying out the Invention

### Formulation optimized for fusion protein dimer

In an aspect of the present invention, there is provided an aqueous pharmaceutical formulation comprising a fusion protein dimer comprising an extracellular domain of an alpha subunit of an IgE Fc receptor (FcεRIα ECD). Here, the pH of the aqueous pharmaceutical formulation may be in the range of 6.0 to 7.0, 6.1 to 7.0, 6.2 to 6.9, 6.3 to 6.8, 6.4 to 6.8, or 6.4 to 6.6.

As used herein, the term "pharmaceutical formulation" refers to a preparation that exists in a form that allows the biological activity of an active ingredient to be clearly effective and does not include an ingredient that causes side effects in a subject to which the formulation is administered.

The term "subject" may be a mammal such as human, dog, cow, horse, pig, sheep, goat, cat, mouse, rabbit, and rat, and may be preferably human, dog, or cat.

As used herein, the term "aqueous pharmaceutical formulation" refers to a pharmaceutical formulation that uses a suitable aqueous solvent, such as water or an aqueous/oily mixture (for example, a mixture of water and alcohol). The formulation may maintain stability, such as chemical or physical stability, or biological activity.

The term "stability" refers to a property that maintains a constant state, and is generally related to minimize degradation, denaturation, aggregation, or unfolding of a biologically active substance, such as a protein, a peptide, or a biologically active macromolecule.

Meanwhile, the formulation may be a liquid formulation. The liquid formulation is an aqueous solution or suspension, and may be stably maintained at room temperature, refrigerated (for example, 2°C to 8°C) or frozen (for example, -20°C or -70°C) during storage.

The aqueous pharmaceutical formulation of the present invention may be administered parenterally. In this case, parenteral administration may be performed by a method such as subcutaneous administration, intravenous administration, mucosal administration, and intramuscular administration. In an embodiment of the present invention, the formulation may be preferably administered by subcutaneous injection.

According to the claims, the fusion protein dimer in the aqueous pharmaceutical formulation may be contained at a high concentration, specifically 50 mg/mL or more, 60 mg/mL or more, or 70 mg/mL or more. In an embodiment of the present invention, the concentration of the fusion protein dimer may be 50 mg/mL to 150 mg/mL, 50 mg/mL to 130 mg/mL, 50 mg/mL to 120 mg/mL, 50 mg/mL to 110 mg/mL, 50 mg/mL to 100 mg/mL, 50 mg/mL to 90 mg/mL, 50 mg/mL to 80 mg/mL, 60 mg/mL to 150 mg/mL, 60 mg/mL to 130 mg/mL, 60 mg/mL to 120 mg/mL, 60 mg/mL to 110 mg/mL, 60 mg/mL to 100 mg/mL, 60 mg/mL to 90 mg/mL, 60 mg/mL to 80 mg/mL, 70 mg/mL to 150 mg/mL, 70 mg/mL to 130 mg/mL, 70 mg/mL to 120 mg/mL, 70 mg/mL to 110 mg/mL, 70 mg/mL to 100 mg/mL, 70 mg/mL to 90 mg/mL or 70 mg/mL to 80 mg/mL, and preferably 75 mg/mL, but is not limited thereto.

In addition, the aqueous pharmaceutical formulation comprises a buffer and a stabilizer. Here, the buffer is histidine. The histidine may be present at a concentration of 10 mM to 100 mM in the formulation. In an embodiment of the present invention, the histidine as a buffer may be present at a concentration of 10 mM to 90 mM, 10 mM to 80 mM, 10 mM to 70 mM, 10 mM to 60 mM, 20 mM to 90 mM, 20 mM to 80 mM, 20 mM to 70 mM, 20 mM to 60 mM, 30 mM to 90 mM, 30 mM to 80 mM, 30 mM to 70 mM, 30 mM to 60 mM, 40 mM to 90 mM, 40 mM to 80 mM, 40 mM to 70 mM, 40 mM to 60 mM, 50 mM to 90 mM, 50 mM to 80 mM, 50 mM to 70 mM, or 50 mM to 60 mM in the formulation. Preferably, it may be present at a concentration of 55 mM.

The histidine buffer may comprise a solution of histidine chloride, histidine acetate, histidine phosphate, histidine sulfate, but is not limited thereto. The pH of the histidine buffer or histidine-HCl buffer may be in the range of 5.5 to 7.5, 6.0 to 7.0, 6.1 to 7.0, 6.2 to 6.9, 6.3 to 6.8, 6.4 to 6.8, or 6.4 to 6.6, and preferably may be pH 6.5.

The stabilizer is proline. The proline may be present at a concentration of 200 mM to 300 mM in the formulation. In an embodiment of the present invention, the proline may be present at a concentration of 200 mM to 290 mM, 200 mM to 280 mM, 200 mM to 270 mM, 200 mM to 260 mM, 210 mM to 290 mM, 210 mM to 280 mM, 210 mM to 270 mM, 210 mM to 260 mM, 220 mM to 290 mM, 220 mM to 280 mM, 220 mM to 270 mM, 220 mM to 260 mM, 230 mM to 290 mM, 230 mM to 280 mM, 230 mM to 270 mM, 230 mM to 260 mM, 240 mM to 290 mM, 240 mM to 280 mM, 240 mM to 270 mM, or 240 mM to 260 mM in the formulation. Preferably, it may be present at a concentration of 250 mM.

The aqueous pharmaceutical formulation of the present invention may further comprise an antioxidant. Here, the antioxidant may be methionine. The methionine may be present at a concentration of 10 mg/mL to 30 mg/mL in the formulation. In an embodiment of the present invention, the methionine may be present at a concentration of 10 mg/mL to 28 mg/mL, 10 mg/mL to 26 mg/mL, 10 mg/mL to 24 mg/mL, 10 mg/mL to 22 mg/mL, 12 mg/mL to 28 mg/mL, 12 mg/mL to 26 mg/mL, 12 mg/mL to 24 mg/mL, 12 mg/mL to 22 mg/mL, 14 mg/mL to 28 mg/mL, 14 mg/mL to 26 mg/mL, 14 mg/mL to 24 mg/mL, 14 mg/mL to 22 mg/mL, 16 mg/mL to 28 mg/mL, 16 mg/mL to 26 mg/mL, 16 mg/mL to 24 mg/mL, 16 mg/mL to 22 mg/mL, 18 mg/mL to 28 mg/mL, 18 mg/mL to 26 mg/mL, 18 mg/mL to 24 mg/mL, or 18 mg/mL to 22 mg/mL in the formulation. Preferably, it may be present at a concentration of 20 mg/mL.

The antioxidant is an agent that inhibits the oxidation of other molecules and may reduce the formation of aggregates in the formulation by removing oxygen.

In addition, the aqueous pharmaceutical formulation may further comprise a surfactant. The surfactant is an agent that lowers the surface tension of a liquid and may control the formation of soluble and insoluble aggregates. The surfactant may be a non-ionic surfactant, and may be polysorbates such as polysorbate 20, polysorbate 28, polysorbate 40, polysorbate 60, polysorbate 65, polysorbate 80, polysorbate 81, and polysorbate 85; poloxamers such as poloxamer 181, poloxamer 188, and poloxamer 407; or polyethylene glycol (PEG) but is not limited thereto. Preferably, the surfactant may be poloxamer.

Specifically, the poloxamer may be poloxamer 188. The poloxamer 188 may be present at a concentration of 0.01 w/v% to 1 w/v% in the formulation. In an embodiment of the present invention, the poloxamer 188 may be present at a concentration of 0.02 w/v% to 0.8 w/v%, 0.02 w/v% to 0.6 w/v%, 0.02 w/v% to 0.4 w/v%, 0.02 w/v% to 0.2 w/v%, 0.02 w/v% to 0.15 w/v%, 0.04 w/v% to 0.8 w/v%, 0.04 w/v% to 0.6 w/v%, 0.04 w/v% to 0.4 w/v%, 0.04 w/v% to 0.2 w/v%, 0.04 w/v% to 0.15 w/v%, 0.06 w/v% to 0.8 w/v%, 0.06 w/v% to 0.6 w/v%, 0.06 w/v% to 0.4 w/v%, 0.06 w/v% to 0.2 w/v%, 0.06 w/v% to 0.15 w/v%, 0.08 w/v% to 0.8 w/v%, 0.08 w/v% to 0.6 w/v%, 0.08 w/v% to 0.4 w/v%, 0.08 w/v% to 0.2 w/v%, 0.08 w/v% to 0.15 w/v%, 0.09 w/v% to 0.8 w/v%, 0.09 w/v% to 0.6 w/v%, 0.09 w/v% to 0.4 w/v%, 0.09 w/v% to 0.2 w/v%, or 0.09 w/v% to 0.15 w/v% in the formulation. Preferably, it may be present at a concentration of 0.1 w/v%.

In another aspect of the present invention, there is provided an aqueous pharmaceutical formulation comprising i) a fusion protein dimer comprising FcεRIα ECD at a concentration of 50 mg/mL to 150 mg/mL; ii) histidine at a concentration of 10 mM to 100 mM; iii) proline at a concentration of 200 mM to 300 mM; iv) methionine at a concentration of 10 mg/mL to 30 mg/mL; and v) poloxamer 188 at a concentration of 0.01 w/v% to 1 w/v%. Here, the pH of the aqueous pharmaceutical formulation may be in the range of 6.0 to 7.0, 6.1 to 7.0, 6.2 to 6.9, 6.3 to 6.8, 6.4 to 6.8, or 6.4 to 6.6. Preferably, it may be pH 6.5.

In an embodiment of the present invention, the aqueous pharmaceutical formulation may comprise i) a fusion protein dimer comprising FcεRIα ECD at a concentration of 75 mg/mL; ii) histidine at a concentration of 55 mM; iii) proline at a concentration of 250 mM; iv) methionine at a concentration of 20 mg/mL; and v) poloxamer 188 at a concentration of 0.1 w/v%.

The aqueous pharmaceutical formulation may be stored in a container selected from the group consisting of a vial, a cartridge, a syringe, and an autoinjector.

In addition, the container in which the formulation is stored may be stored at room temperature, at 2°C to 8°C, or at 25°C to 40°C until administered to a subject in need of treatment.

The formulation may be administered by parenteral administration such as subcutaneous administration, intravenous administration, mucosal administration, intramuscular administration, or intraperitoneal administration, but not limited thereto, using an 18G to 32G needle in a volume of 5 mL or less, 3 mL or less, or 2 mL or less. Preferably, it may be subcutaneously administered using a 27G needle in a volume of 1 mL or less.

### Fusion protein comprising extracellular domain of alpha subunit of IgE Fc receptor

The fusion protein dimer described above is as follows. Specifically, the dimer comprises two monomers, each of which comprises the extracellular domain of the alpha subunit of the IgE Fc receptor (FcεRIα ECD).

The monomer may comprise a modified Fc region, and the modified Fc region and FcεRIα ECD may be linked via a hinge of an IgD antibody.

As used herein, the term "IgE" means an antibody protein known as immunoglobulin E. IgE has affinity to mast cells, basophils in blood, or the like. In addition, reaction between an IgE antibody and an antigen (allergen) corresponding thereto causes an inflammatory reaction. In addition, IgE is known to be an antibody that causes anaphylaxis which occurs due to sudden secretion of mast cells or basophils.

As used herein, the term "IgE Fc receptor" is also referred to as Fcε receptor, and binds to an Fc portion of IgE. There are two types for the receptors. The receptor having high affinity to IgE Fc is called Fcε receptor I (FcεRI). The receptor having low affinity to IgE Fc is called Fcε receptor II (FcεRII). FcεRI is expressed in mast cells and basophils. In a case where IgE antibodies bound to FcεRI are cross-linked by polyvalent antigens, degranulation occurs in mast cells or basophils, thereby releasing various chemical transmitter substances including histamine. This release leads to an immediate allergic reaction.

The FcεRI is a membrane protein composed of one α chain, one β chain, and two γ chains linked by a disulfide bond. Among these chains, a portion to which IgE binds is the α chain (FcεRIα), and FcεRIα has a size of about 60 kDa, and is composed of a hydrophobic domain existing inside the cell membrane and a hydrophilic domain existing outside the cell membrane. In particular, IgE binds to the extracellular domain of the α chain.

Specifically, the alpha subunit of the IgE Fc receptor may have the amino acid sequence set forth in NP_ 001992.1. In addition, the extracellular domain of the alpha subunit of the IgE Fc receptor (FcεRIα ECD) may have the amino acid sequence of SEQ ID NO: 1. In the present specification, the extracellular domain of the alpha subunit of the IgE Fc receptor may be a fragment or variant of the extracellular domain of the alpha subunit of the IgE Fc receptor, as long as the fragment or variant is capable of binding to IgE.

The variant may be prepared through a method of substituting, deleting, or adding one or more proteins in the wild-type FcεRIα ECD (extracellular domain), as long as the method does not alter a function of the α chain of FcεRI. Such various proteins or peptides may be 90%, 91%, 92%, 93%, 94%, 95%, 96%, 97%, 98% or 99% or more identical to the amino acid sequence of SEQ ID NO: 1. In addition, FcεRIα ECD of SEQ ID NO: 1 may be encoded by a polynucleotide having the sequence of SEQ ID NO: 5.

In addition, as used herein, the term "modified Fc region" means a region in which a part of the Fc portion of an antibody has been modified. Here, the term "Fc region" refers to a protein which contains heavy chain constant region 2 (CH2) and a heavy chain constant region 3 (CH3) of an immunoglobulin and does not contain variable regions of heavy and light chains, and light chain constant region 1 (CH1) of an immunoglobulin. In particular, the modified Fc region means a region obtained by substituting some amino acids in the Fc region or by combining different types of Fc regions. Specifically, the modified Fc region may have the amino acid sequence of SEQ ID NO: 2. In addition, the modified Fc region of SEQ ID NO: 2 may be encoded by a polynucleotide having the sequence of SEQ ID NO: 6.

In addition, the "modified Fc region" of the present invention may be in the form of having sugar chains in a native form, increased sugar chains relative to a native form, or decreased sugar chains relative to the native form, or may be in the form of being sugar chain-removed. Immunoglobulin Fc sugar chains may be modified by conventional methods such as chemical methods, enzymatic methods, and genetic engineering methods using microorganisms.

Here, the "modified Fc region" of the present invention may be a region that lacks antibody dependent cellular cytotoxicity (ADCC) and complement dependent cytotoxicity (CDC) functions due to having no binding site for FcyR or C1q. In addition, the modified Fc region and FcεRIα ECD may be linked via a hinge of an IgD antibody. The hinge of the IgD antibody is composed of 64 amino acids and may selectively include 20 to 60 consecutive amino acids, or 25 to 50 consecutive amino acids, or 30 to 40 amino acids. In an embodiment, the hinge of the IgD antibody may be composed of 30 or 49 amino acids as shown below. In addition, the hinge of the IgD antibody may be a hinge variant obtained by modifying the hinge region, in which the hinge may include at least one cysteine. Here, the hinge variant may be obtained by modifying some in a hinge sequence of the IgD antibody in order to minimize the generation of truncated forms during a protein production process.

In an embodiment, the hinge may include the following sequence:
Arg Asn Thr Gly Arg Gly Gly Glu Glu Lys Lys Xaa1 Xaa2 Lys Glu Lys Glu Glu Gln Glu Glu Arg Glu Thr Lys Thr Pro Glu Cys Pro (SEQ ID NO: 17), wherein Xaa1 may be Lys or Gly, and Xaa2 may be Glu, Gly or Ser. Specifically, the hinge may have the amino acid sequences of SEQ ID NO: 3 or SEQ ID NO: 19, thereby minimizing the generation of truncated forms during a protein production process.

In another embodiment, the hinge may include the following sequence:
Ala Gln Pro Gln Ala Glu Gly Ser Leu Ala Lys Ala Thr Thr Ala Pro Ala Thr Thr Arg Asn Thr Gly Arg Gly Gly Glu Glu Lys Lys Xaa3 Xaa4 Lys Glu Lys Glu Glu Gln Glu Glu Arg Glu Thr Lys Thr Pro Glu Cys Pro (SEQ ID NO: 18), wherein Xaa3 may be Lys or Gly, and Xaa4 may be Glu, Gly or Ser. Specifically, the hinge may have the amino acid sequence of SEQ ID NO: 4, thereby minimizing the generation of truncated forms during a protein production process.

In particular, at least one of Thr in the hinge having the sequence of SEQ ID NO: 4 may be glycosylated. Specifically, the 13^{th}, 14^{th}, 18^{th}, and 19^{th} Thr in the amino acids of SEQ ID NO: 18 may be glycosylated. Preferably, all four amino acids may be glycosylated. Here, the glycosylation may be O-glycosylation.

In addition, as described above, the fusion protein dimer provided by the present invention may be in a form in which two monomers are bound to each other and each monomer is obtained by binding between an extracellular domain of an alpha subunit of an IgE Fc receptor and a modified Fc region. The fusion protein dimer may be in a form in which the same two monomers are bound to each other by cysteine located at a hinge site. In addition, the fusion protein dimer may be in a form in which two different monomers are bound to each other. For example, in a case where the two monomers are different form each other, the polypeptide dimer may be in a form in which one monomer includes the extracellular domain of the alpha subunit of the IgE Fc receptor, and the other monomer includes a fragment of the extracellular domain of the alpha subunit of the IgE Fc receptor. Here, an embodiment of the monomer may have the amino acid sequence of SEQ ID NO: 20, SEQ ID NO: 21, or SEQ ID NO: 22.

In addition, the fusion protein dimer provided by the present invention exhibit a binding affinity to IgE which is 10- to 100-fold, 20- to 90-fold, 20- to 70-fold, 30- to 70-fold, or 40- to 70-fold higher than Omalizumab, an anti-IgE antibody, and may preferably exhibit a binding affinity to IgE which is 70-fold higher than Omalizumab.

### Therapeutic use of formulation optimized for fusion protein dimer

**In** another aspect of the present invention, there is provided the aqueous pharmaceutical formulation comprising the fusion protein dimer for use in the prevention or treatment of allergic diseases.

In the present specification, the term "allergic disease" means a pathological symptom caused by an allergic reaction mediated by mast cell activation, such as mast cell degranulation. Such allergic diseases include food allergy, atopic dermatitis, asthma, allergic rhinitis, allergic conjunctivitis, allergic dermatitis, allergic contact dermatitis, anaphylaxis, urticaria, pruritus, insect allergy, chronic idiopathic urticaria, chronic spontaneous urticaria, drug allergy, and the like. In particular, the allergic diseases may be IgE-mediated.

In the aqueous pharmaceutical formulation of the present invention may be administered to a subject according to a dosage regimen and dosage effective for treating allergic diseases. The fusion protein dimer in the formulation of the present invention may be contained in any amount (effective amount), as long as the active ingredient can exhibit an anti-allergic activity. A typical effective amount of the active ingredient will be determined within a range of 0.001% by weight to 20.0% by weight based on a total weight of the composition. Here, the "effective amount" refers to an amount capable of inducing an anti-allergic effect. Such an effective amount can be determined experimentally within the ordinary skill of those skilled in the art.

A preferable daily dosage of the formulation is ranged from 0.01 µg/kg to 10 g/kg, and preferably 0.01 mg/kg to 1 g/kg, depending on the patient's condition, body weight, sex, age, disease severity, or route of administration. Administration may be carried out once or several times a day. Such a dosage should in no way be interpreted as limiting the scope of the present invention.

Disclosed, but not claimed, is a method for treating or preventing allergic diseases, comprising administering to a subject the aqueous pharmaceutical formulation comprising the fusion protein dimer.

The subject may be a mammal, preferably a human. In this case, administration may be performed through a parenteral route. In this case, parenteral administration may be performed by subcutaneous administration, intravenous administration, mucosal administration, intramuscular administration, or the like.

### Mode for Carrying out the Invention

Hereinafter, the present invention will be described in more detail with reference to the following examples. However, the following examples are intended to merely illustrate the present invention, and the scope of the present invention is not limited thereto.

### Preparation Example 1. Preparation of fusion protein dimer comprising FcεRIα ECD and modified Fc region: GI-301

A C-terminal modified polypeptide of the extracellular domain of the alpha subunit of the IgE Fc receptor (FcεRIα-ECD) was prepared according to the method disclosed in US Patent No. 7,867,491.

First, in order to express the protein (FcεRIaECD-Fc1), a protein (FcεRIαECD-Fc2), and a protein (FcεR1αECD-Fc3), in which the extracellular domain of the α chain of FcεRI having the amino acid sequence of SEQ ID NO: 1 and the modified immunoglobulin Fc of SEQ ID NO: 2 are linked via the hinge of SEQ ID NO: 19, the hinge of SEQ ID NO: 3, and the hinge of SEQ ID NO: 4, respectively, cassettes obtained by linking the gene encoding each protein were cloned into a vector to construct the FcεRIα ECD-Fc protein expression vector. Then, each of the expression vectors was transfected into CHO cells.

Here, at the time of being transduced into the cell line, the expression vector obtained by cloning an α-2,6-sialic acid transferase gene into a vector was simultaneously transfected to separately prepare cell lines which are capable of expressing FcεRIα ECD-Fc2ST and FcεRIα ECD-Fc3ST proteins to which sialic acid is added. The fusion protein dimer produced from the cell line was named "GI-301."

### Example 1. Optimization of buffer and pH conditions for production of highly concentrated GI-301

The test was conducted to develop a liquid formulation for subcutaneous injection including about 100 mg/mL of a GI-301 substance. The formulation should be stable for storage at 2°C to 8°C and the viscosity of the formulation should be low enough for injection. First, the buffer and pH conditions were optimized.

To determine an appropriate formulation buffer, a total of 25 different buffers with different pH, buffer components, ionic strength and stabilizers were selected by design of experiments (DOE), and pre-screening (step 1 and step 2) and final screening (step 3) were performed.
i) Pre-screening (step 1 and step 2):
   - Dynamic laser light scattering was measured in a DLS plate reader to confirm the increase in colloidal and thermodynamic stability of the sample.
   - The sample was dialyzed against the formulation buffer for each condition.
   - Colloidal stability was determined by confirming the protein-protein interaction by measuring the hydrodynamic radius of the sample for each concentration in each formulation buffer. These results were used to predict formulation candidates with a low risk of protein aggregation during handling and storage.
   - The denaturation temperature (thermodynamic stability) was determined by measuring the hydrodynamic radius of the sample for each temperature in each formulation buffer. These results were used to predict formulation candidates with a low risk of protein denaturation during handling and storage. When the protein domain begins to denature, the hydrodynamic radius of the protein significantly increases. The onset-temperature of unfolding can be regarded as an indicator of the secondary structure stability of protein.
ii) Final screening (step 3):
   - Colloidal stability (intermolecular interaction, A2) was measured by CG-MALS (composition gradient multi-angle light scattering).
   - Thermodynamic stability was measured by Nano DSC (nano differential scanning calorimetry).
   - The aggregation state of protein was confirmed by SE-HPLC.
   - Relative potency was measured by ELISA.
   - According to the above results, the four most suitable formulations were selected.

Specifically, in the first 14 samples in Table 1, the general physical properties of the GI-301 regarding pH, buffer components and ionic strength were evaluated. Samples were prepared by dialysis of GI-301 against each formulation buffer.

The pH was adjusted from 6.0 to 7.4, which is an acceptable pH range for subcutaneous injection. For each pH value, suitable buffer components were selected to achieve sufficient buffer capacity. The buffer components were selected from pharmaceutically acceptable excipients. Sodium chloride was added to adjust the ionic strength of the formulation (maximum ionic strength is set to isotonic). The results of the first pre-screening are summarized in Table 1 below.

**[Table 1] (Samples 1-14 are not according to the claims)**

| **#** | **pH** | **buffer component** | **salt/stabilizer** | **Tₒₙₛₑₜ** [°C] | **kd[mL/mg×10⁻²]¹)** | **actual pH** |
|---|---|---|---|---|---|---|
| 1 | 7.4 | 10 mM Na-phosphate | 150 NaCl | 65 | -0.1 | 7.36 |
| 2 | 7.4 | 10 mM Na-phosphate | w/o | 68 | 3.1 | 7.36 |
| 3 | 7.4 | 10 mM Tris/HCl | 150 NaCl | 65 | -0.1 | 7.40 |
| **4** | **7.4** | **10 mM Tris/HCl** | **w/o** | **64** | **5.7** | **7.38** |
| 5 | 6.7 | 10 mM Na-phosphate | 150 NaCl | 66 | -1.2 | 6.72 |
| 6 | 6.7 | 10 mM Na-phosphate | w/o | 60 | 3.3 | 6.68 |
| 7 | 6.7 | 10 mM His/HCl | 150 NaCl | 63 | -1.1 | 6.71 |
| **8** | **6.7** | **10 mM His/HCl** | **w/o** | **69** | **14.2** | **6.53** |
| 9 | 6.0 | 10 mM His/HCl | 150 NaCl | 54 | -1.4 | 6.03 |
| **10** | **6.0** | **10 mM His/HCl** | **w/o** | **59** | **5.8** | **6.01** |
| 11 | 6.7 | 10 mM Na-citrate | 150 NaCl | 65 | -1.2 | 6.62 |
| 12 | 6.7 | 10 mM Na-citrate | w/o | 66 | 0.6 | 6.66 |
| 13 | 6.0 | 10 mM Na-citrate | 150 NaCl | 64 | -1.4 | 6.00 |
| 14 | 6.0 | 10 mM Na-citrate | w/o | 63 | 0.1 | 6.00 |

| | | | | | | |
|---|---|---|---|---|---|---|
| 1) Example for sample #1: kd = -0.1 mL/mg × 10⁻² = -0.001 mL/mg | | | | | | |

In general, the colloidal stability of GI-301 is lower at high ionic strength and is independent of the used buffer system and the pH of the formulation. At low ionic strength, the buffer component showed the greatest influence on the colloidal stability of GI-301. The influence of pH was not significant. The best colloidal stability was observed in the His/HCl buffer system, followed by Tris/HCl, sodium phosphate and sodium citrate. In general, more acidic pH values of the formulations lead to lower colloidal stability in one buffer group. The excellent colloidal stability was observed in formulation sample #8 (10 mM His/HCl, pH 6.7), sample #10 (10 mM His/HCI, pH 6.0) and sample #4 (10 mM Tris/HCl, pH 7.4).

The thermodynamic stability of GI-301 was similar in all formulation samples except samples #9 and #10. GI-301 exhibited high thermodynamic stability at generally used drug storage temperatures.

The actual pH values of the dialyzed samples met the desired pH values except formulation sample #8, in which a slight Donnan-effect was observed.

In consideration of these results, His/HClat pH 6.7 and Tris/HCl at pH 7.4 were selected as the most appropriate buffers for GI-301. Stabilizers and non-ionic isotonic agents suitable for liquid and/or lyophilization were tested in samples #15 to #20. The excipients used in the tests were trehalose (a cryoprotectant used in lyophilization), mannitol (a bulking agent used in lyophilization and a non-ionic isotonic agent) and proline (an amino acid stabilizer used in liquid formulations, non-ionic). The results of the two-step screening for excipients are summarized in Table 2 below.

**[Table 2] (Samples 15,16,18-20 are not according to the claims)**

| # | **pH** | **buffer component** | **salt/stabilizer** | **Tₒₙₛₑₜ** [°C] | **kd[mL/mg×10⁻²]** | **actual pH** |
|---|---|---|---|---|---|---|
| 15 | 6.7 | 10 mM His/HCl | 250 mM trehalose | 67 | 6.3 | 6.44 |
| **16** | **6.7** | **10 mM His/HCl** | **250 mM mannitol** | **66** | **13.5** | **6.52** |
| **17** | **6.7** | **10 mM His/HCl** | **250 mM proline** | **64** | **20.4** | **6.55** |
| 18 | 7.4 | 10 mM Tris/HCl | 250 mM trehalose | 65 | 5.2 | 7.34 |
| **19** | **7.4** | **10 mM Tris/HCl** | **250 mM mannitol** | **68** | **6.1** | **7.40** |
| **20** | **7.4** | **10 mM Tris/HCl** | **250 mM proline** | **68** | **7.3** | **7.39** |

An increased colloidal stability was observed upon addition of mannitol and proline in both buffer systems His/HCland Tris/HCl. A slightly decreased colloidal stability was observed upon addition of trehalose. The excipients did not exhibit remarkable influences neither on the thermodynamic stability of GI-301 nor on the actual pH.

In the final screening experiment, the most promising formulation samples for liquids and lyophilizates were investigated using CG-MALS (A₂), Nano DSC (Tₒₙₛₑₜ) SE-HPLC (relative monomer content) and ELISA (relative potency). The results of the final screening are summarized in Table 3 below.

**[Table 3] (Samples 21,22,24,25 are not according to the claims)**

| **#** | **pH** | **buffer component** | **stabilizer** | **Tₒₙₛₑₜ Nano DSC [°C]** | **A₂ [10⁻⁴ mol*ml*g⁻²]** | **relative monomer content by SE-HPLC [%]** | **potency ELISA [%]** |
|---|---|---|---|---|---|---|---|
| 21 | 6.7 | 10 mM His/HCl | 250 mM trehalose | 51 | 4.8 | 96.2 | 103 |
| 22 | 6.7 | 10 mM His/HCl | 250 mM mannitol | 52 | 7.8 | 96.1 | 98 |
| 23 | 6.7 | 10 mM His/HCl | 250 mM proline | 52 | 8.7 | 96.1 | 101 |
| 24 | 7.4 | 10 mM Tris/HCl | 250 mM mannitol | 51 | 4.4 | 96.1 | 92 |
| 25 | 7.4 | 10 mM Tris/HCl | 250 mM proline | 49 | 4.8 | 96.1 | 106 |

The relative ranking of the five samples for colloidal stability and Tₒₙₛₑₜ was similar to the previous results obtained with DLS. CG-MALS results showed strong repulsive protein-protein-interaction in all formulation samples. Tₒₙₛₑₜ of thermal unfolding was lower in the nanoDSC measurement compared to the DLS measurement. DLS measures the hydrodynamic radius increases of the protein, which is a result of aggregation due to thermal unfolding. In contrast, nanoDSC measured the thermodynamic transition from folded protein to unfolded protein and thus showed lower values for Tₒₙₛₑₜ. The relative potency as measured by ELISA was nearly 100% in all formulation samples.

Based on the above results, a "concentration challenge test" was performed in consideration of the items described in Table 4 below.

**[Table 4] (Samples 21,22,25 are not according to the claims)**

| **#** | **pH** | **buffer component** | **stabilizer** | **description** |
|---|---|---|---|---|
| 21 | 6.7 | 10 mM His/HCl | 250 mM trehalose | Liquid formulation with actual pH 6.5; back-up for a freeze-dried presentation |
| 22 | 6.7 | 10 mM His/HCl | 250 mM mannitol | Liquid formulation with pH 6.5; commonly used excipients |
| 23 | 6.7 | 10 mM His/HCl | 250 mM proline | Liquid formulation with pH 6.5; may have lower tendency for aggregation |
| 25 | 7.4 | 10 mM Tris/HCl | 250 mM proline | Liquid formulation with pH 7.4; perfect isotonic conditions, but may have less chemical stability |

### Example 2. Test on the possibility of high concentration of GI-301

Among the 25 samples tested in Example 1, the four most appropriate samples were selected for the concentration challenge test. The four candidate formulation samples selected based on the results of Example 1 are summarized in Table 5 below.

**[Table 5] (Samples 1,2,4 are not according to the claims)**

| # | **pH** | **buffer component** | **stabilizer** |
|---|---|---|---|
| 1 | 6.7 | 10 mM His/HCl | 250 mM trehalose |
| 2 | 6.7 | 10 mM His/HCl | 250 mM mannitol |
| 3 | 6.7 | 10 mM His/HCl | 250 mM proline |
| 4 | 7.4 | 10 mM Tris/HCl | 250 mM proline |

The above four formulation candidates were tested as follows:
- It was confirmed whether the target concentration of about 100 mg/mL was achieved in each formulation sample (using ultrafiltration and process-related time interval).
- The pH values of the concentrated and mixed samples were measured to monitor the pH shift due to the Donnan-effect.
- The samples were filtered using a 0.22 µm membrane filter and aseptically filled into a 2R vial using a multi-pipette with sterile plunger tips (target fill volume of 1.2 mL for 100 mg/mL).
- Challenge tests were performed on all highly concentrated samples by storage at 4°C and 35°C for 72 hours.
- After 72 hours, the samples were visually analyzed for turbidity and precipitation.
- Viscosity and syringeability: the syringeability of each formulation sample was evaluated by measuring the push-out force. The contents of the syringes were dispensed through the attached needle with a defined traverse speed using a force/displacement measuring device. The samples were analyzed for the following items: visual appearance, concentration by UV 280, pH, viscosity using a capillary viscometer, turbidity by nephelometric measurement, osmolality, relative potency by ELISA, and aggregation state by SE-HPLC.

As a result, the four formulation samples in Table 5 above could be successfully concentrated to a concentration of 100 mg/mL. The highly viscous (100 mg/mL) solution in the vial did not show precipitation or appreciable turbidity after storage at 35°C for 72 hours.

According to the syringeability test, it was confirmed that the usage of 30G cannula led to the push-out force exceeding 20 N limit for all 100 mg/mL samples. When the 27G cannula was used, the push-out force was acceptable. When it was diluted to a concentration of 50 mg/mL, the 30G cannula was acceptable.

As a result of concentration, the pH was decreased by about 0.4 to 0.8 (Donnan-effect). To compensate the Donnan effect, the pH values were adjusted to the original concentration using highly concentrated L-histidine and TRIS base.

The final protein concentration was about 65 mg/mL. 1 mL syringe filled with 1 mL of the sample was pushed through a tensile machine and the shear-stressed material (content pushed out of the syringe) was analyzed.

All results are shown in Table 6 below.

**[Table 6]**

| | | | **Sample** | | | |
|---|---|---|---|---|---|---|
| **Concentration of GI-301** | **Primary packaging material** | **Method** | **#1** | **#2** | **#3** | **#4** |
| 100 mg/mL | 2R vial | Visual appearance / precipitation | √ | √ | √ | √ |
| | | After 72 hours at 5°C | | | | |
| 100 mg/mL | 2R vial | Visual appearance / precipitation | √ | √ | √ | √ |
| | | After 72 hours at 35°C | | | | |
| 100 mg/mL | 1 mL syringe 30G cannula | Push-out force | X | X | X | X |
| | | Visual appearance / precipitation | √ | √ | √ | √ |
| 100 mg/mL | 1 mL syringe 27G cannula | Push-out force | √ | √ | √ | √ |
| | | Visual appearance / precipitation | √ | √ | √ | √ |
| 50 mg/mL | 1 mL syringe 30G cannula | Push-out force | √ | √ | √ | √ |
| | | Visual appearance / precipitation | √ | √ | √ | √ |
| | | pH - influence of push-out | √ | √ | √ | √ |
| 65 mg/mL (Donnan effect; compensated for sample #2 - #4) | 1 mL syringe 27G cannula | Push-out force | | √ | √ | √ |
| | | Visual appearance / precipitation | | √ | √ | √ |
| | | pH - influence of push-out | | √ | √ | √ |
| | | SEC | | √ | √ | √ |
| | | ELISA | | √ | √ | √ |
| | | Viscosity | | √ | √ | √ |
| | | Turbidity | | √ | √ | √ |
| | | Osmolality | | √ | √ | √ |

| | | | | | | |
|---|---|---|---|---|---|---|
| *Acceptable results:* √ *Unacceptable results: X* | | | | | | |

In the case of using the protein at a concentration of 65 mg/mL or less, the syringeability was confirmed for all four formulation samples even with the 30G cannula.

The GI-301 protein was very stable during short-term storage at a concentration of 100 mg/mL or less in all four formulation samples.

Since long-term stability cannot be predicted based on the short-term data, an accelerated stability test was additionally performed.

The four formulation candidates showed similar results as shown in Table 6 above.

### Example 3. Accelerated stability test (Forced degradation test I)

In Example 2, the protein could be concentrated at a high concentration in the four formulation buffers, and the syringeability was also verified. As a result of analysis of the four samples, it was confirmed that there were no remarkable differences between them. Accordingly, the forced degradation test was additionally performed on the four samples of Example 2 (see Table 5) using a 2R vial.

The four formulation candidates with a target protein concentration of 50 mg/mL were prepared, and the following steps were performed:
- Filling aseptically ten 2R vials per sample
- Forced thermal stress and agitation: for 2 weeks or for 5 days at 200 rpm at 35 °C
- Exposure to light: for 7.5 hours under a condition of 750 W/m²
- The samples were analyzed for the following items: visual appearance, concentration by UV 280, pH, turbidity by nephelometric measurement, osmolality, relative potency by ELISA, and aggregation state by SE-HPLC.

The four formulation samples were successfully concentrated to a concentration of about 50 mg/mL with an adequate pH shift compensation. Thereafter, an accelerated stress stability test was performed using 2R vials.

The best (√) and worst (X) results are shown in Table 7 below.

**[Table 7]**

| **Experiment method** | | **Sample** | | | | | | | | | | | | | | | |
|---|---|---|---|---|---|---|---|---|---|---|---|---|---|---|---|---|---|
| | | **#1** | | | | **#2** | | | | **#3** | | | | **#4** | | | |
| Visual inspection | | 5 days, 35°C, 200 rpm | 14 days, 35°C, 200 rpm | | light | 5 days, 35°C, 200 rpm | 14 days, 35°C, 200 rpm | | light | 5 days, 35°C, 200 rpm | 14 days, 35°C, 200 rpm | | light | 5 days, 35°C, 200 rpm | 14 days, 35°C, 200 rpm | | light |
| | | X | X | | √ | √ | X | | √ | √ | X | | √ | √ | X | | √ |
| SE-HPLC | | 14 days, 35°C, 200 rpm | | light | | 14 days, 35°C, 200 rpm | | light | | 14 days, 35°C, 200 rpm | | light | | 14 days, 35°C, 200 rpm | | light | |
| SE-HP LC | Main peak (general) | | | | | | | | | √ | | √ | | X | | X | |
| | Aggregates | | | | | | | | | √ | | √ | | X | | X | |
| | Fragments | √ | X | | | | | | | | | | | | | X | √ |

According to the results, sample #3 (proline, histidine/HCl, pH 6.7) was the most suitable buffer for the next development step. In addition, this sample was used to perform the 150 mg/mL concentration test via cross-flow filtration.

Meanwhile, as a result of SE-HPLC analysis, all formulation samples could be frozen/thawed three times without significant negative effect.

### Example 4. Feasibility analysis of high concentration via cross-flow filtration

Based on the results of Example 3, the formulation comprising 250 mM proline, 10 mM histidine/HCl, pH 6.7 was selected as a formulation for feasibility analysis of high concentration (150 mg/mL) via cross-flow filtration. The objective is to obtain a concentrated protein solution (150 mg/mL).

### Example 4.1. Cross-flow filtration and pH adjustment

In the first step, 1,400 g of 7.5 mg/mL substance was concentrated to a volume of about 200 mL. Concentration was performed at a trans-membrane pressure (TMP) of 1.0 bar.

After this step, buffer exchange (diafiltration each) was started in an isovolumetric manner.

The permeate drain was substituted with the target buffer, which was fed into stirred container of the cross-flow device. The total fed amount of the target buffer was 950 g. The concentration of the original DS buffer was below 1% at the end of the diafiltration step.

Then, the second concentration step was carried out until a volume of about 110 g was reached. The pH target of 6.70 was adjusted with 150 mM histidine/250 mM proline buffer. Thereafter, the concentration step was continued.

It was confirmed that the maximal concentration was reached when the permeate volume decreased and the cross-flow filtration device sounded the peak pressure alarm. The pH was finely adjusted, and a concentrate was harvested. The obtained solution was pre-filtered with a 1 µm filter, filtered with a 0.2 µm filter under sterile conditions, and stored at 2°C to 8°C until further processing.

### Example 4.2. Visual inspection

After diafiltration and concentration, the solution was slightly yellowish and showed turbidity.

The solution obtained after filtration with 1.0 µm and 0.2 µm filters was clear and slightly yellowish.

### Example 4.3. UV 280

The concentration of the obtained solution was 106.3 mg/mL±2.6 mg/mL after filtration (n=3).

### Example 4.4. Syringeability

Table 8 shows the maximal push-out force during the measurements. The 27G cannula was suitable for GI-301 with a concentration of about 100 mg/mL. The 30G cannula exhibited too high push-out force at a concentration of about 100 mg/mL. Each of the above experiments was repeated twice (n=2).

**[Table 8]**

| Cannula | Fₘₐₓ [N] |
|---|---|
| 27G (0.4 mm OD) 12 mm | 10.6±0.5 |
| 30G (0.3 mm OD) 12 mm | 23.5±1.1 |

It was confirmed that cross-flow concentration of GI-301 was feasible. The target concentration (150 mg/mL) could not be achieved due to the high viscosity of the solution at high concentration. The concentration after diafiltration was about 106 mg/mL. The syringeability was demonstrated as moderate push-out forces of about 11 N with a 27G cannula.

### Example 5. Accelerated stability test (Forced degradation test II)

In Example 4, the buffer composition (250 mM proline, histidine/HCl, pH 6.7) was concentrated to a maximal concentration of about 106 mg/mL via cross-flow filtration. Accordingly, forced degradation test II was conducted with a target concentration of GI-301 of 100 mg/mL. A surfactant and/or an antioxidant methionine was added to sample #3 obtained in Example 2 to confirm the advantages of these additives (Table 9). 2R vials were filled with 1.5 mL of the corresponding solution and the samples were analyzed after thermal/mechanical stress and light stress exposure.

**[Table 9]**

| Basic composition | 250 mM proline, histidine/HCl, pH 6.7 | | | | | |
|---|---|---|---|---|---|---|
| **Additive** | **#1** (no additive) | **#2** | **#3** | **#4** | **#5¹⁾** | **#6¹⁾** |
| Methionine | | 20 mg/mL | | 20 mg/mL | 20 mg/mL | 20 mg/mL |
| Tween 80 | | | 0.1 w/v% | 0.1 w/v% | | |
| Poloxamer | | | | | 0.1 w/v% | |
| Tween 20 | | | | | | 0.1 w/v% |

| | | | | | | |
|---|---|---|---|---|---|---|
| ¹⁾ Samples were prepared after obtaining #1 to #4 results. Formulation samples for forced degradation test with surfactant and methionine are as follows: #1: Formulation without additives (no PS80, no methionine) #2: Formulation comprising methionine (20 mg/mL) #3: Formulation comprising Tween 80 (0.1 w/v%) #4: Formulation comprising Tween 80 (0.1 w/v%) and methionine (20 mg/mL) #5: Formulation comprising poloxamer 188 (0.1 w/v%) and methionine (20 mg/mL) #6: Formulation comprising Tween 20 (0.1 w/v%) and methionine (20 mg/mL) | | | | | | |

### Example 5.1. Visual inspection

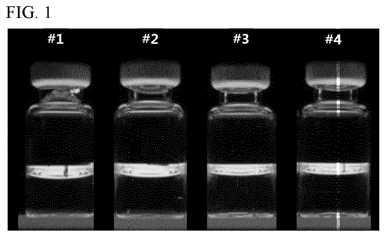

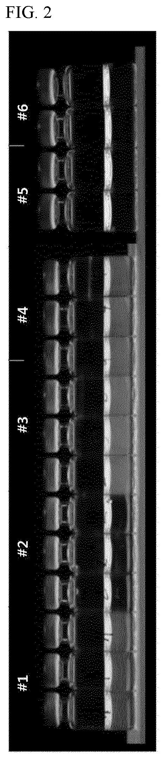

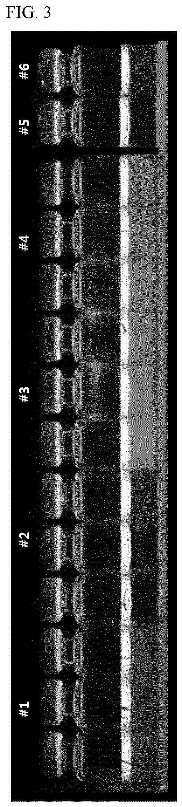

As a result of visual inspection, the solution was more yellowish due to light stress. After storage for 14 days at 35°C and 200 rpm, turbidity was increased in samples #1, #3 and #4, slight turbidity was shown in sample #6, and samples #2 and #5 showed a clear solution state (FIGS. 1 to 3). Table 10 shows the results of visual inspection of each sample.

**[Table 10]**

| | Sample | | | | | |
|---|---|---|---|---|---|---|
| | #1 | #2 | #3 | #4 | #5 | #6 |
| light stress 7.5 hours, 750 W/m² | clear solution, yellowish | clear solution, yellowish | clear solution, yellowish | clear solution, yellowish | not applicable | not applicable |
| 35°C, 200 rpm, 5 days | visible turbidity, pale yellowish | clear solution, pale yellowish | visible turbidity, pale yellowish | visible turbidity, pale yellowish | clear solution, pale yellowish | clear solution, pale yellowish |
| 35°C, 200 rpm, 14 days | strong turbidity, pale yellowish | clear solution, pale yellowish | strong turbidity, pale yellowish | strong turbidity, pale yellowish | clear solution, pale yellowish | slight turbidity, pale yellowish |

### Example 5.2. UV 280

Table 11 shows the concentrations of the samples after the forced degradation test. There were no remarkable differences according to stress exposure.

**[Table 11]**

| | Sample (n=2) | | | | | |
|---|---|---|---|---|---|---|
| | #1 | #2 | #3 | #4 | #5 | #6 |
| | c [mg/mL] | c [mg/mL] | c [mg/mL] | c [mg/mL] | c [mg/mL] | c [mg/mL] |
| 35°C, 200 rpm, 5 days | 96.8±0.7 | 98.9±2.1 | 96.2±0.3 | 95.5±0.3 | 93.9±1.5 | 95.3±2.8 |
| 35°C, 200 rpm, 14 days | 102.1±1.6 | 94.3±1.7 | 96.5±2.5 | 94.5±1.5 | 95.6±1.0 | 96.0±2.5 |
| light stress 7.5 hours 750 W/m² | 97.6±1.6 | 96.1±1.1 | 95.2±0.8 | 95.8±2.4 | - | - |

### Example 5.3. pH measurement

Table 12 shows the pH of the samples after the forced degradation test. There were no remarkable changes in pH.

**[Table 12]**

| | Sample (n=2) | | | | | |
|---|---|---|---|---|---|---|
| | #1 | #2 | #3 | #4 | #5 | #6 |
| | pH | pH | pH | pH | pH | pH |
| 35°C, 200 rpm, 5 days | 6.82±0.01 | 6.74±0.00 | 6.74±0.00 | 6.72±0.00 | not applicable | not applicable |
| 35°C, 200 rpm, 14 days | 6.76±0.00 | 6.71±0.00 | 6.73±0.01 | 6.70±0.00 | not applicable | not applicable |
| light stress 7.5 hours 750 W/m² | 6.72±0.01 | 6.72±0.00 | 6.71±0.01 | 6.70±0.01 | not applicable | not applicable |

### Example 5.4. Size exclusion chromatography (SE-HPLC)

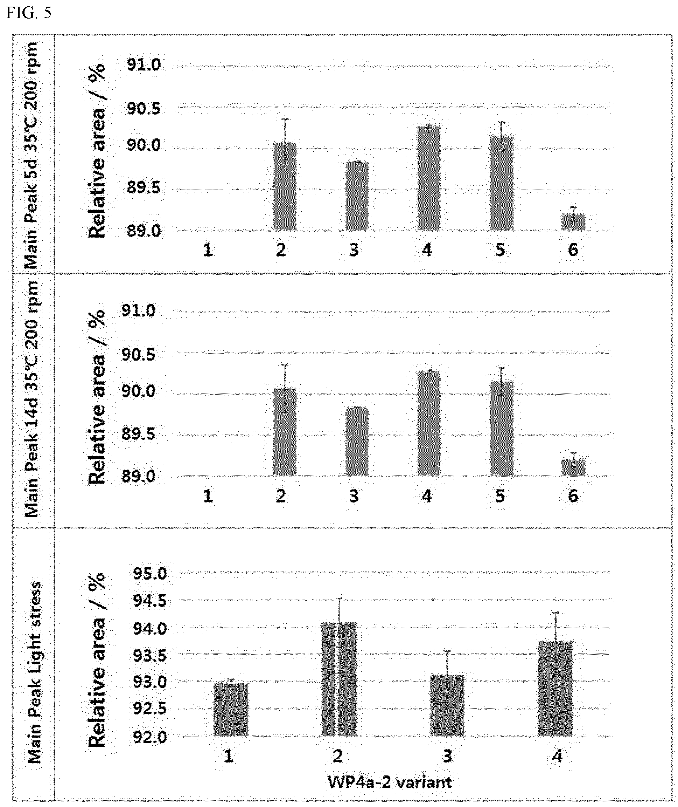

SE-HPLC is a standard method for the detection of aggregated and fragmented proteins. The results of SEC measurement are shown in Table 13 and FIGS. 5 to 7.

**[Table 13]**

| Sample (n=2) | | | | | | |
|---|---|---|---|---|---|---|
| stress type | #1 | #2 | #3 | #4 | #5 | #6 |
| main peak area/percent (%) | | | | | | |
| 35°C, 200 rpm, 5 days | 53.15±1.14 | 90.07±0.29 | 89.84±0.00 | 90.27±0.02 | 90.15±0.17 | 89.20±0.08 |
| 35°C, 200 rpm, 14 days | 36.83±0.08 | 88.07±0.05 | 87.52±0.04 | 87.89±0.05 | 87.78±0.03 | 86.19±0.01 |
| light stress | 92.97±0.07 | 94.08±0.45 | 93.12±0.43 | 93.74±0.52 | not applicable | not applicable |

| aggregates area/percent (%) | | | | | | |
|---|---|---|---|---|---|---|
| 35°C, 200 rpm, 5 days | 5.78±0.23 | 8.20±0.08 | 8.72±0.04 | 8.24±0.06 | 8.26±0.11 | 9.23±0.05 |
| 35°C, 200 rpm, 14 days | 4.90±0.07 | 9.38±0.06 | 9.85±0.05 | 9.42±0.01 | 9.46±0.05 | 10.97±0.01 |
| light stress | 6.06±0.03 | 5.28±0.00 | 6.26±0.02 | 5.63±0.06 | not applicable | not applicable |

| fragments area/percent (%) | | | | | | |
|---|---|---|---|---|---|---|
| 35°C, 200 rpm, 5 days | 41.07±1.37 | 1.73±0.21 | 1.44±0.03 | 1.50±0.04 | 1.59±0.05 | 1.58±0.13 |
| 35°C, 200 rpm, 14 days | 58.27±0.01 | 2.55±0.01 | 2.63±0.01 | 2.69±0.05 | 2.77±0.02 | 2.84±0.01 |
| light stress | 0.98±0.04 | 0.64±0.45 | 0.62±0.45 | 0.63±0.46 | not applicable | not applicable |

| recovery/percent (%) | | | | | | |
|---|---|---|---|---|---|---|
| 35°C, 200 rpm, 14 days | 97.17±0.26 | 100.25±0.07 | 98.61±0.14 | 98.56±0.04 | 100.17±0.14 | 100.43±0.12 |

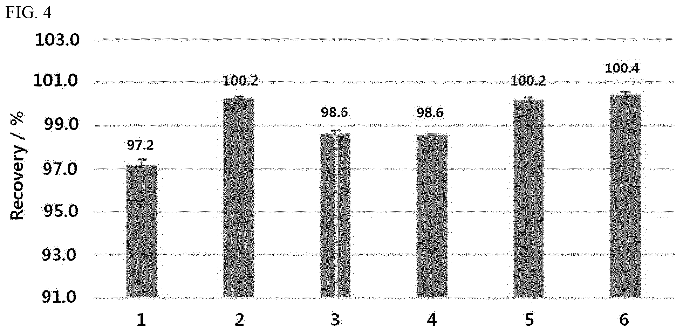

As shown in Table 13, the recovery rate under the stress condition of 35°C and 200 rpm for 14 days, was comparable to the visual inspection: samples #2, #5 and #6 showed high recovery rate (FIG. 4).

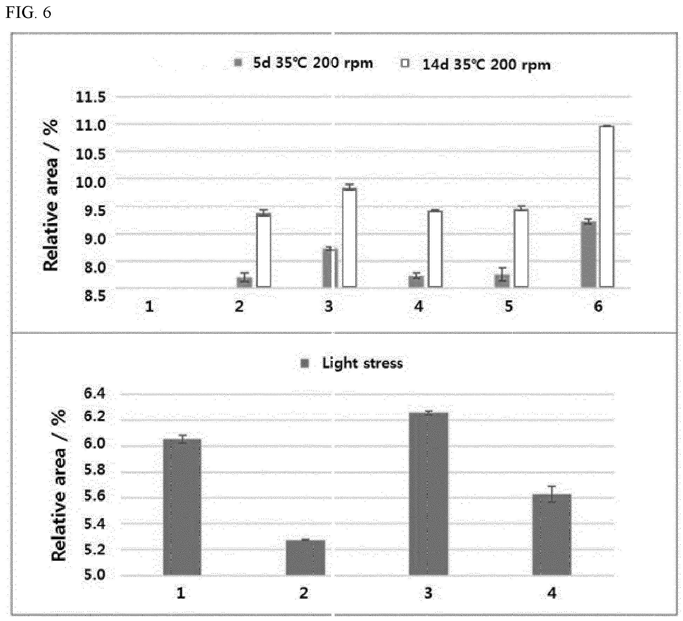

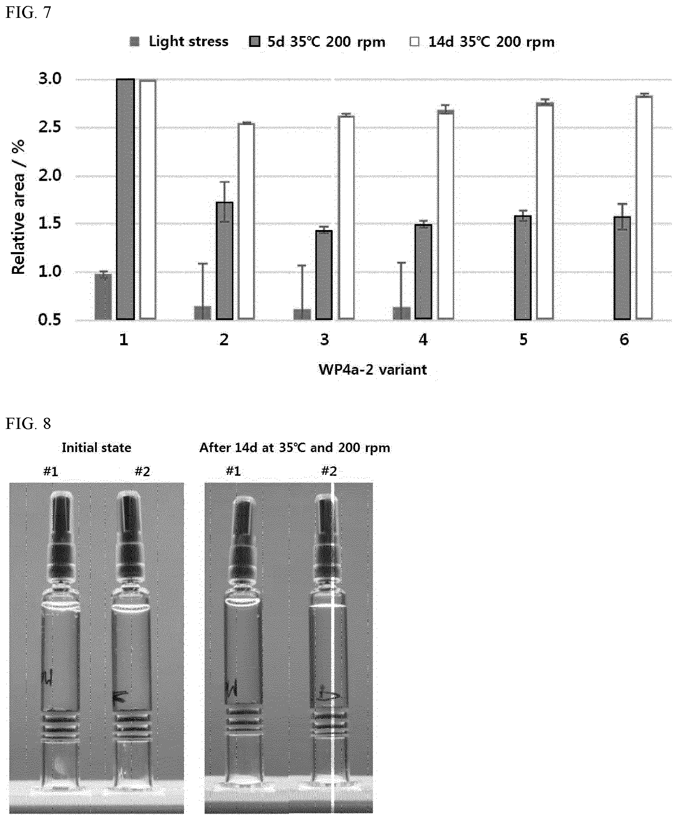

In addition, as shown in FIG. 5, as a result of measuring the main peak, better results were obtained in samples #2, #4 and #5 to which thermal/mechanical stress was applied, and samples #1 and #6 showed poor results. Meanwhile, as shown in FIG. 6, protein aggregation was showed in sample #6. Under the light stress condition, sample #2 showed the least amount of protein aggregation. As shown in FIG. 7, sample #2 showed the best results with respect to the protein fragment.

### Example 5.5. ELISA-based Potency analysis of GI-301

The relative potency of GI-301 was determined according to the results of ELISA-based potency analysis. The principle of this analysis is to quantify GI-301 molecules capable of binding to human IgE.

The relative potency analyzed by ELISA is shown in Table 14. All samples maintained high binding affinity.

**[Table 14]**

| | Sample (n=2) | | | |
|---|---|---|---|---|
| | #1 | #2 | #3 | #4 |
| 35°C, 200 rpm, 5 days | 79.7%±3.8% | 112.7%±1.6% | 92.1%±5.2% | 94.3%±30.4 |
| 35°C, 200 rpm, 14 days | 104.9%±0.0% | 74.2%±6.5% | 80.4%±7.5% | 110.9%±12.6% |
| light stress | 110.2%±6.6% | 112.2%±5.1% | 110.1%±6.8% | 111.2%±3.3% |

### Example 5.6. Turbidity by nephelometric measurement

Table 15 shows the results of the turbidity measurements. There was no clear correlation between visual appearance and turbidity measurements (compare samples #3 and #4 after storage at 35°C for 5 days). A potential cause of this discrepancy might be the increased opalescence in the concentrated protein solution, which increases the base turbidity. As a result, the visible turbidity can higher than the basic turbidity due to the protein aggregation. Therefore, the samples were stored at 35°C for 14 days and then analyzed by DLS.

**[Table 15]**

| | Sample (n=2) | | | |
|---|---|---|---|---|
| | #1 | #2 | #3 | #4 |
| | NTU | NTU | NTU | NTU |
| 35°C, 200 rpm, 5 days | 35.05±0.07 | 11.52±3.80 | 11.50±0.14 | 10.70±0.14 |
| light stress | 11.65±1.48 | 9.38±1.59 | 7.37±0.20 | 6.84±0.06 |

### Example 5.7. Osmolality

The osmolality results are shown in Table 16. As expected, methionine increased osmolality.

**[Table 16]**

| | Sample (n=2) | | | |
|---|---|---|---|---|
| | #1 | #2 | #3 | #4 |
| | mOSMOL/kg | mOSMOL/kg | mOSMOL/kg | mOSMOL/kg |
| 35°C, 200 rpm, 5 days | 351±1 | 500±2 | 338±0 | 498±1 |
| 35°C, 200 rpm, 14 days | 363±1 | 488±3 | 333±1 | 492±0 |
| light stress | 338±2 | 496±1 | 338±1 | 495±0 |

### Example 5.8. Analysis of measurement results

The results are summarized in Table 17 below.

**[Table 17]**

| Sample | #1 | #2 | #3 | #4 | #5 | #6 |
|---|---|---|---|---|---|---|
| Visual appearance after 14 days at 35°C/200 rpm | △ | ○ | × | × | ○ | △ |
| SEC recovery after 14 days at 35°C/200 rpm | × | ○ | △ | △ | ○ | ○ |
| SEC main peak after 14 days at 35°C/200 rpm | × | ○ | △ | ○ | ○ | × |
| DLS after 14 days at 35°C/200 rpm | △ | △ | × | × | △ | △ |

| | | | | | | |
|---|---|---|---|---|---|---|
| ×: poor result, △: moderate result, ○: good result Based on the results, the most suitable formulation samples are as follows: Sample #2 : His/proline pH 6.7; 20 mg/mL methionine Sample #5 : His/proline pH 6.7; 20 mg/mL methionine; 0.1 w/v% poloxamer 188 | | | | | | |

As a result of the test, sample #2 and sample #5 were suitable among the formulation samples in Table 17 above.

### Example 6. Forced degradation test using syringe

Two formulations were selected by Example 5 using a 2R vial: 250 mM proline, histidine/HCl, pH 6.7, 0.1 w/v% poloxamer 188 with/without, 20 mg/mL methionine (antioxidant). Based on these results, an antioxidant and a surfactant were added to the samples (Table 18). The target concentration of the sample was 100 mg/mL. 2.25 mL syringes were filled with 1.75 mL of the corresponding solution and the samples were analyzed after thermal/mechanical stress exposure for 14 days.

The samples for forced degradation study are as follows:
#1: Formulation comprising methionine (20 mg/mL)
#2: Formulation comprising poloxamer 188 (0.1 w/v%) and methionine (20 mg/mL)

**[Table 18]**

| Basic ingredients | 250 mM proline, histidine/HCl, pH 6.7 | |
|---|---|---|
| additive | #1 | #2 |
| methionine | 20 mg/mL | 20 mg/mL |
| poloxamer 188 | - | 0.1 w/v% |

### Example 6.1. Visual inspection

The results of visual inspection are shown in Table 19. As shown in FIG. 8, there were no noticeable differences after storage at 35°C for 14 days.

**[Table 19]**

| | Sample | |
|---|---|---|
| | #1 | #2 |
| initial state | clear solution, yellowish | clear solution, yellowish |
| 35°C, 200 rpm, 14 days | clear solution, yellowish | clear solution, yellowish |

### Example 6.2. Measurement of push-out force

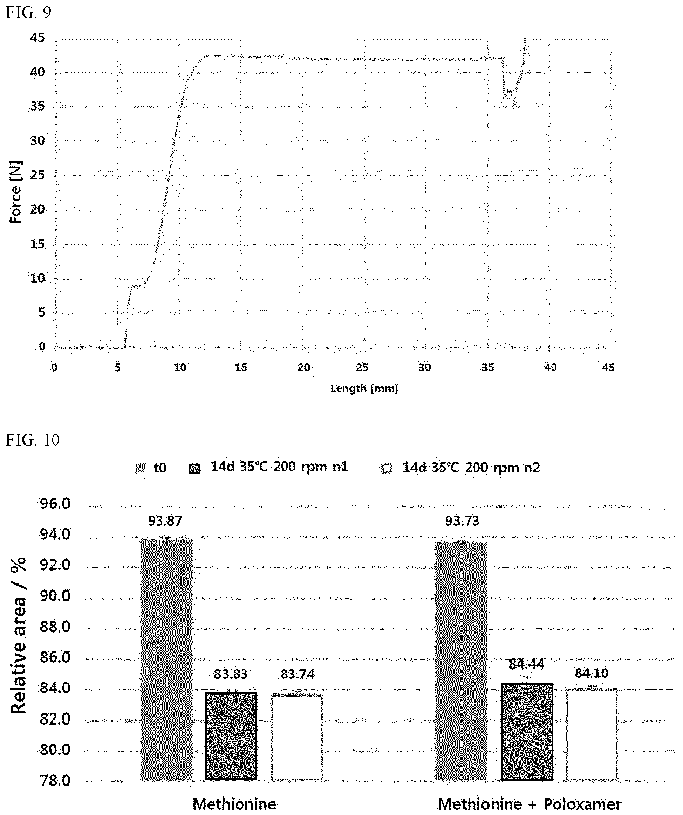

Table 20 shows results of the tensile machine. A push-out force exceeding 20 N occurred in both samples when the 27G cannula was used (FIG. 9). Therefore, a syringe with a larger inner diameter or a larger needle size (for example, 25G) should be selected to tolerate the protein with 100 mg/mL concentration.

**[Table 20]**

| 27G (0.4 mm OD) 18 mm | Sample | |
|---|---|---|
| 27G (0.4 mm OD) 18 mm | #1 | #2 |
| 27G (0.4 mm OD) 18 mm | Fₘₐₓ[N] | Fₘₐₓ[N] |
| n1 | 43.7 | 43.3 |
| n2 | 42.6 | 41.0 |

### Example 6.3. UV 280

Table 21 shows the concentrations of the samples after the forced degradation test. There were no remarkable differences after stress exposure.

**[Table 21]**

| Sample (n=2) | | | | | |
|---|---|---|---|---|---|
| initial state | | after 14 days at 35°C, 200 rpm | | | |
| #1 | #2 | #1 syringe 1 | #1 syringe 2 | #2 syringe 1 | #2 syringe 2 |
| c[mg/mL] | c[mg/mL] | c[mg/mL] | c[mg/mL] | c[mg/mL] | c[mg/mL] |
| 98.9±2.9 | 99.6±1.6 | 100.2±1.2 | 101.3±0.8 | 98.2±1.0 | 98.9±0.7 |

### Example 6.4. pH measurement

Table 22 shows the pH of the samples after the forced degradation test. There were no remarkable changes in pH.

**[Table 22]**

| Sample (n=2) | | | | | |
|---|---|---|---|---|---|
| initial state | | after 14 days at 35°C, 200 rpm | | | |
| #1 | #2 | #1 syringe 1 | #1 syringe 2 | #2 syringe 1 | #2 syringe 2 |
| pH | pH | pH | pH | pH | pH |
| 6.74±0.00 | 6.77±0.01 | 6.75±0.01 | 6.73±0.03 | 6.75±0.00 | 6.75±0.00 |

### Example 6.5. Size exclusion chromatography (SE-HPLC)

The results of the SEC measurement are shown in Table 23 and FIG. 10.

**[Table 23]**

| Sample (n=2) | | | | | |
|---|---|---|---|---|---|
| initial state (t0) | | after 14 days at 35°C, 200 rpm | | | |
| #1 | #2 | #1 syringe 1 | #1 syringe 2 | #2 syringe 1 | #2 syringe 2 |
| main peak area/percent (%) | | | | | |
| 93.87±0.14 | 93.73±0.03 | 83.83±0.04 | 83.74±0.16 | 84.44±0.41 | 84.10±0.11 |
| aggregates area/percent (%) | | | | | |
| 4.36±0.04 | 4.49±0.01 | 9.60±0.04 | 9.61±0.08 | 9.40±0.16 | 9.48±0.05 |
| fragments area/percent (%) | | | | | |
| 1.76±0.18 | 1.79±0.02 | 6.57±0.00 | 6.66±0.08 | 6.16±0.26 | 6.41±0.06 |
| recovery/percent (%) | | | | | |
| 98.19±0.02 | 98.79±0.03 | 101.56±0.28 | 102.99±0.34 | 101.44±0.72 | 102.72±0.14 |

After storage at 35°C and 200 rpm for 14 days, the difference between the two samples was insignificant. However, the better results of the main peak area were shown in sample #2 (with poloxamer) compared to sample #1 (without poloxamer). Compared to the results with vials, the fragments area increased by about 4%. This was consistent with the main peak results (FIG. 10).

### Example 6.6. Osmolality

The osmolality results are shown in Table 24. Osmolality was maintained at the same level even after stress exposure.

**[Table 24]**

| Sample (n=2) | | | | | |
|---|---|---|---|---|---|
| initial state | | after 14 days at 35°C, 200 rpm | | | |
| #1 | #2 | #1 syringe 1 | #1 syringe 2 | #2 syringe 1 | #2 syringe 2 |
| mOSMOL/kg | mOSMOL/kg | mOSMOL/kg | mOSMOL/kg | mOSMOL/kg | mOSMOL/kg |
| 504±1 | 507±2 | 515±8 | 514±6 | 514±2 | 516±1 |

### Example 6.7. Sub-visible particles

The amount of sub-visible particles was evaluated using a fluid imaging microscope. The stressed syringes filled with water had a low particle burden. This indicates that the influence of a siliconized syringe on a particle burden is limited.

Sample #1 and sample #2 showed a comparable particle burden. Most of the particles observed were silicone particles, and no particles indicating protein aggregates/precipitation.

### Example 6.8. Analysis of measurement results

The results are summarized in Table 25.

**[Table 25]**

| Sample | #1 | #2 |
|---|---|---|
| Visual appearance after 14 days at 35°C/200 rpm | O | ○ |
| SEC recovery after 14 days at 35°C/200 rpm | O | ○ |
| SEC main peak after 14 days at 35°C/200 rpm | ○ | ○ |
| DLS after 14 days at 35°C/200 rpm | Δ | Δ |
| Flow imaging microscope (qualitative) | O | ○ |

| | | |
|---|---|---|
| ×: poor result, Δ: moderate result, ○: good result | | |

Both samples showed almost similar results. The only difference is that sample #2 showed slightly better results in SEC analysis (relative main peak area).

According to the above results, the buffer formulation of 250 mM proline, 10 mM histidine/HCl, pH 6.7 comprising GI-301 at a concentration of 100 mg/mL, 20 mg/mL methionine as an antioxidant, and 0.1 w/v% poloxamer 188 as a surfactant was the most suitable formulation for GI-301.

However, in the case of the above conditions, the pH was decreased lower than the target pH 6.7 due to the Donnan-effect that occurred in the ultrafiltration process to meet the final concentration of 100 mg/mL. To compensate for the decrease in pH, a process of adjusting the pH by high-concentration histidine dilution after ultrafiltration was added. Finally, 75 mg/mL GI-301, 20 mg/mL methionine, 250 mM proline, 55 mM histidine/HCl, 0.1 w/v% poloxamer 188, pH 6.5 were established as the formulation condition for GI-301.

## Claims

1. An aqueous pharmaceutical formulation comprising:
a fusion protein dimer at a concentration of 50 mg/mL to 150 mg/mL, wherein the fusion protein dimer comprises two monomers, each of which comprises an extracellular domain of an alpha subunit of an IgE Fc receptor (FcεRIα ECD),
a buffer, wherein the buffer is histidine, and
a stabilizer, wherein the stabilizer is proline,
wherein, the pH of the formulation is from 6.0 to 7.0,
wherein the monomer comprises a modified Fc region, and
the modified Fc region and the extracellular domain of the alpha subunit of the IgE Fc receptor are linked via a hinge,
wherein the extracellular domain of the alpha subunit of the IgE Fc receptor consists of the amino acid sequence of SEQ ID NO: 1 or a fragment thereof,
wherein the hinge includes the following sequence Arg Asn Thr Gly Arg Gly Gly Glu Glu Lys Lys Xaa1 Xaa2 Lys Glu Lys Glu Glu Gln Glu Glu Arg Glu Thr Lys Thr Pro Glu Cys Pro (SEQ ID NO: 17), wherein Xaa1 is Lys or Gly, and Xaa2 is Glu, Gly or Ser, or
wherein the hinge includes the following sequence Ala Gln Pro Gln Ala Glu Gly Ser Leu Ala Lys Ala Thr Thr Ala Pro Ala Thr Thr Arg Asn Thr Gly Arg Gly Gly Glu Glu Lys Lys Xaa3 Xaa4 Lys Glu Lys Glu Glu Gln Glu Glu Arg Glu Thr Lys Thr Pro Glu Cys Pro (SEQ ID NO: 18), wherein Xaa3 is Lys or Gly, and Xaa4 is Glu, Gly or Ser, and
wherein the modified Fc region consists of SEQ ID NO: 2.

2. The aqueous pharmaceutical formulation according to claim 1, wherein the formulation is for subcutaneous injection.

3. The aqueous pharmaceutical formulation according to claim 1, wherein:
i) the histidine is at a concentration of 10 mM to 100 mM; or
ii) the proline is at a concentration of 200 mM to 300 mM.

4. The aqueous pharmaceutical formulation according to claim 1, wherein the formulation further comprises an antioxidant, optionally wherein the antioxidant is methionine, further optionally wherein the methionine is at a concentration of 10 mg/mL to 30 mg/mL.

5. The aqueous pharmaceutical formulation according to claim 1, wherein the formulation further comprises a surfactant, optionally wherein the surfactant is poloxamer 188, further optionally wherein the poloxamer 188 is at a concentration of 0.01 w/v% to 1 w/v%.

6. An aqueous pharmaceutical formulation according to claim 1, wherein the formulation further comprises:
methionine at a concentration of 10 mg/mL to 30 mg/mL; and poloxamer 188 at a concentration of 0.01 w/v% to 1 w/v%.

7. An aqueous pharmaceutical formulation according to claim 1, wherein the formulation comprises:
i) the histidine at a concentration of 10 mM to 100 mM;
ii) the proline at a concentration of 200 mM to 300 mM;
iii) methionine at a concentration of 10 mg/mL to 30 mg/mL; and
iv) poloxamer 188 at a concentration of 0.01 w/v% to 1 w/v%.

8. The aqueous pharmaceutical formulation according to claim 6 or 7, wherein the formulation is present in a container selected from the group consisting of a vial, a cartridge, a syringe, and an autoinjector.

9. The aqueous pharmaceutical formulation according to claim 6 or 7, wherein the formulation is for subcutaneous administration.

10. The aqueous pharmaceutical formulation according to claim 6, wherein the formulation is for use in the prevention or treatment of an allergic disease.

11. The aqueous pharmaceutical formulation according to claim 7, wherein the formulation is for use in the prevention or treatment of an allergic disease, optionally wherein the allergic disease is one selected from the group consisting of food allergy, atopic dermatitis, asthma, allergic rhinitis, allergic conjunctivitis, allergic dermatitis, chronic idiopathic urticaria, chronic spontaneous urticaria, and allergic contact dermatitis.

## Patentansprüche

1. Wässrige pharmazeutische Formulierung, umfassend:
ein Fusionsproteindimer in einer Konzentration von 50 mg/ml bis 150 mg/ml, wobei das Fusionsproteindimer zwei Monomere umfasst, von denen jedes eine extrazelluläre Domäne einer alpha-Untereinheit eines IgE-Fc-Rezeptors (FcεRIα ECD) umfasst,
einen Puffer, wobei der Puffer Histidin ist, und
einen Stabilisator, wobei der Stabilisator Prolin ist,
wobei der pH-Wert der Formulierung von 6,0 bis 7,0 beträgt,
wobei das Monomer eine modifizierte Fc-Region umfasst und
die modifizierte Fc-Region und die extrazelluläre Domäne der alpha-Untereinheit des IgE-Fc-Rezeptors über ein Gelenk verbunden sind,
wobei die extrazelluläre Domäne der alpha-Untereinheit des IgE-Fc-Rezeptors aus der Aminosäuresequenz von SEQ ID NO: 1 oder einem Fragment davon besteht,
wobei das Gelenk die folgende Sequenz Arg Asn Thr Gly Arg Gly Gly Glu Glu Lys Lys Xaal Xaa2 Lys Glu Lys Glu Glu Gln Glu Glu Arg Glu Thr Lys Thr Pro Glu Cys Pro (SEQ ID NO: 17) einschließt, wobei Xaa1 Lys oder Gly ist und Xaa2 Glu, Gly oder Ser ist, oder
wobei das Gelenk die folgende Sequenz Ala Gln Pro Gln Ala Glu Gly Ser Leu Ala Lys Ala Thr Thr Ala Pro Ala Thr Thr Arg Asn Thr Gly Arg Gly Gly Glu Glu Lys Lys Xaa3 Xaa4 Lys Glu Lys Glu Glu Gln Glu Glu Arg Glu Thr Lys Thr Pro Glu Cys Pro (SEQ ID NO: 18) einschließt, wobei Xaa3 Lys oder Gly ist und Xaa4 Glu, Gly oder Ser ist, und
wobei die modifizierte Fc-Region aus SEQ ID NO: 2 besteht.

2. Wässrige pharmazeutische Formulierung nach Anspruch 1, wobei die Formulierung zur subkutanen Injektion ist.

3. Wässrige pharmazeutische Formulierung nach Anspruch 1, wobei:
i) das Histidin in einer Konzentration von 10 mM bis 100 mM vorliegt; oder
ii) das Prolin in einer Konzentration von 200 mM bis 300 mM vorliegt.

4. Wässrige pharmazeutische Formulierung nach Anspruch 1, wobei die Formulierung ferner ein Antioxidans umfasst, wobei optional das Antioxidans Methionin ist, wobei ferner optional das Methionin in einer Konzentration von 10 mg/ml bis 30 mg/ml vorliegt.

5. Wässrige pharmazeutische Formulierung nach Anspruch 1, wobei die Formulierung ferner ein Tensid umfasst, wobei optional das Tensid Poloxamer 188 ist, wobei ferner optional das Poloxamer 188 in einer Konzentration von 0,01 w/v% bis 1 w/v% vorliegt.

6. Wässrige pharmazeutische Formulierung nach Anspruch 1, wobei die Formulierung ferner Folgendes umfasst:
Methionin in einer Konzentration von 10 mg/ml bis 30 mg/ml; und Poloxamer 188 in einer Konzentration von 0,01 w/v% bis 1 w/v%.

7. Wässrige pharmazeutische Formulierung nach Anspruch 1, wobei die Formulierung Folgendes umfasst:
i) das Histidin in einer Konzentration von 10 mM bis 100 mM;
ii) das Prolin in einer Konzentration von 200 mM bis 300 mM;
iii) Methionin in einer Konzentration von 10 mg/ml bis 30 mg/ml; und
iv) Poloxamer 188 in einer Konzentration von 0,01 w/v% bis 1 w/v%.

8. Wässrige pharmazeutische Formulierung nach Anspruch 6 oder 7, wobei die Formulierung in einem Behälter vorhanden ist, der ausgewählt ist aus der Gruppe bestehend aus einer Ampulle, einer Patrone, einer Spritze und einem Autoinjektor.

9. Wässrige pharmazeutische Formulierung nach Anspruch 6 oder 7, wobei die Formulierung zur subkutanen Verabreichung ist.

10. Wässrige pharmazeutische Formulierung nach Anspruch 6, wobei die Formulierung zur Verwendung bei der Vorbeugung oder Behandlung einer allergischen Erkrankung ist.

11. Wässrige pharmazeutische Formulierung nach Anspruch 7, wobei die Formulierung zur Verwendung bei der Vorbeugung oder Behandlung einer allergischen Erkrankung ist, wobei optional die allergische Erkrankung eine ist, die ausgewählt ist aus der Gruppe bestehend aus Nahrungsmittelallergie, atopischer Dermatitis, Asthma, allergischer Rhinitis, allergischer Konjunktivitis, allergischer Dermatitis, chronischer idiopathischer Urtikaria, chronischer spontaner Urtikaria und allergischer Kontaktdermatitis.

## Revendications

1. Formulation pharmaceutique aqueuse comprenant :
un dimère de protéine de fusion à une concentration de 50 mg/ml à 150 mg/ml, ledit dimère de protéine de fusion comprenant deux monomères, dont chacun comprend un domaine extracellulaire d'une sous-unité alpha d'un récepteur Fc d'IgE (FcεRIα ECD),
un tampon, ledit tampon étant l'histidine, et
un stabilisant, ledit stabilisant étant la proline,
dans laquelle le pH de la formulation est de 6,0 à 7,0,
dans laquelle le monomère comprend une région Fc modifiée, et
la région Fc modifiée et le domaine extracellulaire de la sous-unité alpha du récepteur Fc d'IgE sont liés par l'intermédiaire d'une charnière,
dans laquelle le domaine extracellulaire de la sous-unité alpha du récepteur Fc d'IgE est constitué de la séquence d'acides aminés de SEQ ID N° : 1 ou d'un fragment de celle-ci,
dans laquelle la charnière comprend la séquence suivante Arg Asn Thr Gly Arg Gly Gly Glu Glu Lys Lys Xaa1 Xaa1 Lys Glu Lys Glu Glu Gln Glu Glu Arg Glu Thr Lys Thr Pro Glu Cys Pro (SEQ ID N° : 17), Xaa1 étant Lys ou Gly, et Xaa2 étant Glu, Gly ou Ser, ou
dans laquelle la charnière comprend la séquence suivante Ala Gln Pro Gln Ala Glu Gly Ser Leu Ala Lys Ala Thr Thr Ala Pro Ala Thr Thr Arg Asn Thr Gly Arg Gly Gly Glu Glu Lys Lys Xaa3 Xaa4 Lys Glu Lys Glu Glu Gln Glu Glu Arg Glu Thr Lys Thr Pro Glu Cys Pro (SEQ ID N° : 18), Xaa3 étant Lys ou Gly, et Xaa4 étant Glu, Gly ou Ser, et
dans laquelle la région Fc modifiée est constituée de SEQ ID N° : 2.

2. Formulation pharmaceutique aqueuse selon la revendication 1, ladite formulation étant destinée à une injection sous-cutanée.

3. Formulation pharmaceutique aqueuse selon la revendication 1, dans laquelle :
i) l'histidine est à une concentration de 10 mM à 100 mM ; ou
ii) la proline est à une concentration de 200 mM à 300 mM.

4. Formulation pharmaceutique aqueuse selon la revendication 1, dans laquelle la formulation comprend en outre un antioxydant, éventuellement dans laquelle l'antioxydant est la méthionine, éventuellement en outre dans laquelle la méthionine est à une concentration de 10 mg/ml à 30 mg/ml.

5. Formulation pharmaceutique aqueuse selon la revendication 1, dans laquelle la formulation comprend en outre un tensioactif, éventuellement dans laquelle le tensioactif est le poloxamère 188, en outre éventuellement dans laquelle le poloxamère 188 est à une concentration de 0,01 % p/v à 1 % p/v.

6. Formulation pharmaceutique aqueuse selon la revendication 1, ladite formulation comprenant en outre :
de la méthionine à une concentration de 10 mg/ml à 30 mg/ml ; et du poloxamère 188 à une concentration de 0,01 % p/v à 1 % p/v.

7. Formulation pharmaceutique aqueuse selon la revendication 1, ladite formulation comprenant :
i) l'histidine à une concentration de 10 mM à 100 mM ;
ii) la proline à une concentration de 200 mM à 300 mM ;
iii) de la méthionine à une concentration de 10 mg/ml à 30 mg/ml ; et
iv) du poloxamère 188 à une concentration de 0,01 % p/v à 1 % p/v.

8. Formulation pharmaceutique aqueuse selon l'une des revendications 6 ou 7, ladite formulation étant présente dans un récipient choisi dans le groupe constitué d'un flacon, d'une cartouche, d'une seringue et d'un auto-injecteur.

9. Formulation pharmaceutique aqueuse selon l'une des revendications 6 ou 7, ladite formulation étant destinée à une administration sous-cutanée.

10. Formulation pharmaceutique aqueuse selon la revendication 6, ladite formulation étant destinée à être utilisée dans la prévention ou le traitement d'une maladie allergique.

11. Formulation pharmaceutique aqueuse selon la revendication 7, ladite formulation étant destinée à être utilisée dans la prévention ou le traitement d'une maladie allergique, éventuellement ladite maladie allergique étant une maladie choisie dans le groupe constitué par une allergie alimentaire, la dermatite atopique, l'asthme, la rhinite allergique, la conjonctivite allergique, la dermatite allergique, l'urticaire idiopathique chronique, l'urticaire spontanée chronique et la dermatite de contact allergique.
